# EUROPEAN PATENT APPLICATION

(11) **EP 0 588 546 A2**
(43) Date of publication of application: **23.03.1994**
(21) Application number: 93307042.7
(22) Date of filing: 07.09.1993
(51) Int. Cl.: A61M 25/00, A61M 25/06

(54) **Flexible catheter**

(30) Priority: 08.09.1992 US 941454
(71) Applicant: CRITIKON, INC., Tampa Florida 33634 (US)
(72) Inventor: Van Heugten, Anthony, Tampa, Florida 33635 (US)
(74) Representative: Fisher, Adrian John

(57) **Abstract**

This invention relates to a novel apparatus for improving the performance of an over-the-needle catheter by maintaining constant flow through the catheter. A catheter includes a flexible section. If the patient moves after the catheter is inserted, the flexible section flexes to maintain a constant inner diameter of the catheter. The flexible section is preferably formed by partial compression during molding or extrusion of the catheter.

## Description

### BACKGROUND OF THE INVENTION

### 1. Field of the Invention

This invention relates to a method and apparatus for improving performance of an over-the-needle catheter by using a flexible catheter.

### 2. Description of the Related Art

An over-the-needle catheter is a surgical device for insertion into the tissues of a body cavity. A needle and a concentric outer catheter are inserted into the vein of a patient. After insertion, the needle is withdrawn through the emplaced catheter. Fluids can be introduced or removed through the catheter. The problem of providing constant flow of fluids through a catheter after insertion of the catheter into the body while providing patient comfort during administration of the intended medicament has persisted in the medical community.

A typical catheter assembly is described in U.S. Patent No. 4,747,831 for enhancing patient comfort during insertion of the catheter. In this assembly, a catheter cannula fits snugly, but removably, onto the forwardly projecting part of a needle. The cannula is made of a biologically inert, but very slippery material for ease of insertion of the cannula into the patient. It is disclosed that Teflon is the material used for forming the cannula.

One conventional solution for improving flow of fluids through a catheter while reducing patient discomfort during insertion of the catheter is to use an expandable catheter. U.S. Patent No. 5,061,254 describes a catheter formed of hydrophilic thermoplastic elastomeric polyurethane tubing. A small gauge size catheter can be inserted into the patient. When the catheter comes into contact with a body fluid, such as blood, it absorbs water and expands to a larger gauge size. The larger gauge size of the catheter allows for increased flow of fluids through the catheter.

Prior art catheters have the disadvantage that the catheter can become deformed or kinked if the patient moves after the catheter is inserted in the vein. It is typical to move the patient to change the patient's clothes and linens. Also, with portable intravenous (I.V.) units, the patient may walk around after insertion of the catheter. Patient movement disturbs the site of insertion of the catheter and applies angular pressure to the end of the catheter adjacent to the catheter hub. One conventional solution for preventing movement of the catheter, after insertion of the catheter into the patient, is to elaborately tape the catheter to the arm of the patient. However, even with h taping of the catheter in place, the patient's movements can still deform the catheter.

If the catheter becomes deformed, fluids can be partially or fully restricted from flowing through the catheter. It is often necessary to re-tape the catheter to try to straighten out a deformed catheter. However, reinsertion of the needle into the catheter can cause severance of the catheter. In the alternative, a needle of a replacement over-the-needle catheter can be reinserted into the patient in order to resume flow of fluids to the patient. Reinsertion of the needle causes additional discomfort to the patient.

Of possible general relevance to the invention are U.S. Patent Nos.: 4,850,961; 4,964,854; and 5,000,740 which describe assemblies for over-the-needle catheters.

A practical solution to the problem of maintaining constant flow through a catheter after insertion of the catheter is not found in the prior art.

### SUMMARY OF THE INVENTION

Briefly described, the present invention relates to a flexible catheter for improving the performance of an over-the-needle catheter. A flexible section of the catheter is preferably positioned at the end of the catheter adjacent to the catheter hub. Preferably, the flexible section is formed by partial compression of the catheter during molding or extrusion of the catheter, thereby forming ridges in the catheter. Upon angular movement of the catheter, the portion of the catheter between the ridges expands to provide support to the catheter and to maintain a constant inner diameter of the catheter. An elastic material can be used to form the flexible section for allowing the catheter to return to a straightened position after flexing of the flexible section. The flexible catheter can flex up to an angle of 180° from the point of insertion into the patient. The flexible catheter has the advantage of increasing performance of the catheter by maintaining constant flow of fluids through the catheter.

### BRIEF DESCRIPTION OF THE DRAWINGS

Fig. 1 is a side elevational view of a prior art over-the-needle catheter.
Fig. 2 is a side elevational view of an over-the-needle catheter assembly of the present invention.
Fig. 3 is a cross sectional view of the catheter of the present invention shown in Fig. 2.
Fig. 4 is a side elevational view of flexible portion of the catheter of the present invention.
Fig. 5 is a side elevational view of the catheter of the present invention after insertion of the catheter into the arm of a patient.

### DETAILED DESCRIPTION OF THE INVENTION

During the course of this description like numbers will be used to identify like elements according to the different figures which illustrate the invention.

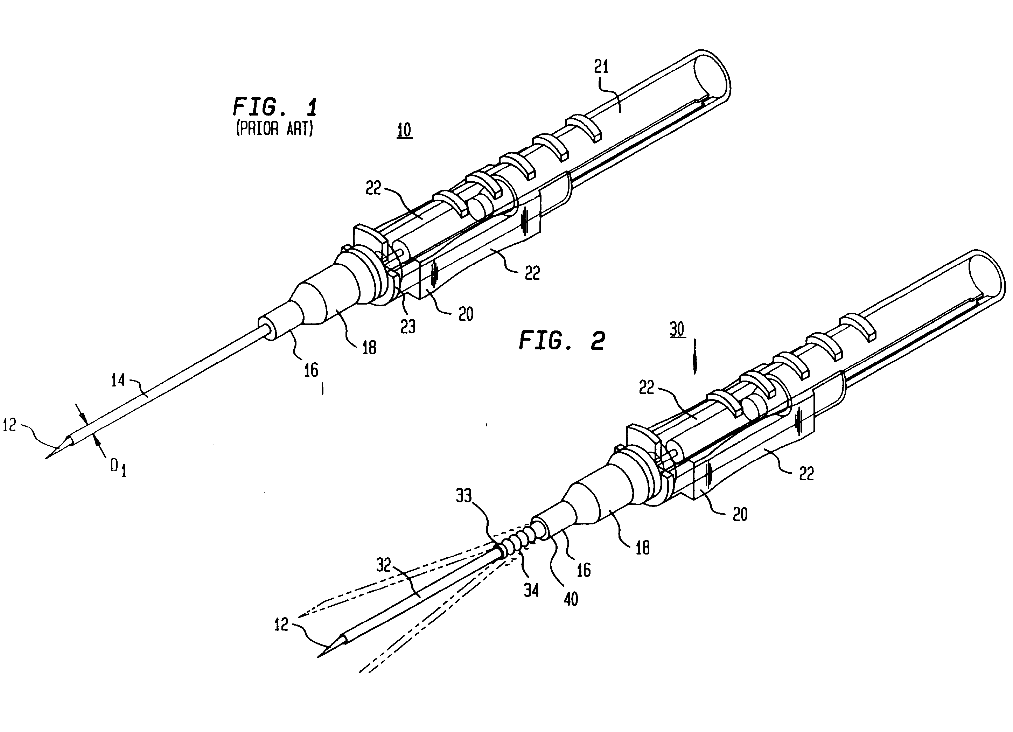

Fig. 1 is a side elevational view of a prior art catheter assembly 10. Catheter 10 can be of the type described in U.S. Patent No. 5,000,740. Catheter 10 includes a needle housing 20 which is semi-tubular in shape. Molded on the sides of needle housing 20 are finger grips 22. At a distal end of catheter housing 20, catheter 14 extends from catheter hub 16 and is concentric therewith. Preferably, catheter hub 16 is hollow. A larger diameter proximal portion 18 of catheter hub 16 is coupled to needle housing 20. Needle 12 is concentric with catheter 14 and can be moved between a use position 23 for inserting needle 12 into the patient and a storage position 21 in housing 20 after needle 12 is inserted into a patient. A point of needle 12 extends from catheter 14 during insertion of catheter 14 into the patient. After insertion of the catheter, catheter 14 can be bent or deformed if the patient moves. When catheter 14 is bent or deformed, inner diameter D₁ of catheter 14 is reduced.

Fig. 2 illustrates a catheter assembly 30 in accordance with the principals of the present invention. Catheter 32 includes flexible section 34. Preferably, flexible section 34 is positioned at the end 33 of catheter 32 adjacent to catheter hub 16. The length of flexible section 34 is dependent on the length of catheter 32 wherein a longer catheter will have a longer flexible section. The length of flexible section 34 is typically defined as about 0.1 inches to about 5.0 inches and the length of catheter 32 is generally about 0.5 inches to about 10 inches. It will be appreciated that the length of the flexible section and the length of the catheter can vary depending on the intended use of the catheter.

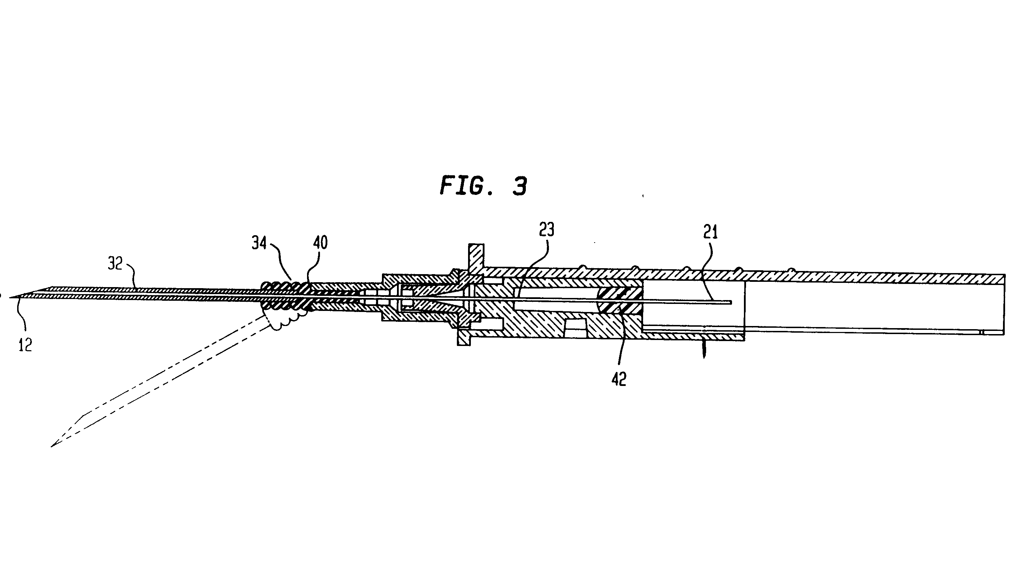

Catheter 32 is preferably attached to catheter hub 16 by means of a metal eyelet 40 to provide a compression fit between catheter 32 and catheter hub 16, as shown in Fig. 3. In the alternative, catheter 32 can be attached by an adhesive to catheter hub 16. It will be appreciated that other means of attachment of the catheter to the catheter hub can be used. Alternatively, catheter 32 can be integral with catheter hub 16.

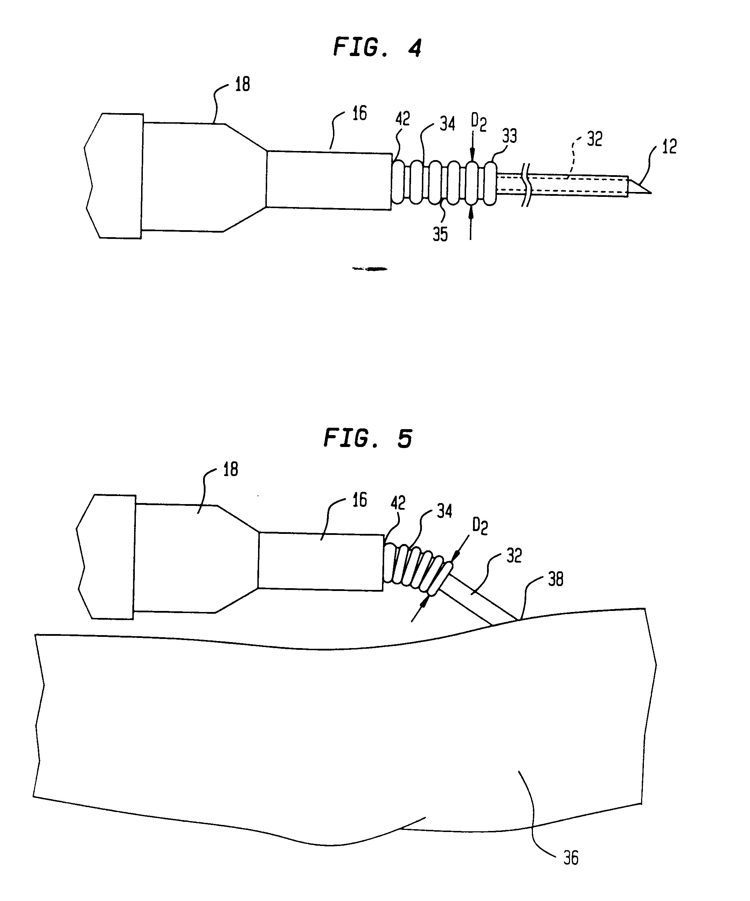

Flexible section 34 is preferably formed by partial compression of catheter 32 during molding or extrusion of the catheter. In the alternative, flexible section 34 can be formed as a subsequent operation after forming catheter 38. Heat can be applied to catheter 32 for aiding compression of the catheter. After compression, ridges 33 are formed in flexible section 34. Upon angular movement of end 42 of catheter 32, ridges 33 and the portion of the catheter 35 positioned between ridges 33 expands. Ridges 33 provide support to flexible section 34 without collapsing inner diameter D₂ of flexible section 34, as shown in Fig. 4.

Catheter 32 can be formed of an elastic or a ductile material. An elastic material allows catheter 32 to return to a straightened position after flexing of flexible section 34. A ductile material allows catheter 32 to remain in a fixed position after flexing of flexible section 34. Preferably, catheter 32 is a hollow tubular molded polyurethane resin. In the alternative, catheter 32 can be formed ofTefion@, which is a registered trademark of Dupont, Dover, Delaware. It will be appreciated to those skilled in the art that other materials could be used for forming catheter 32.

Fig. 5 illustrates catheter assembly 30 after insertion into patient 36 at insertion site 38. Flexible section 34 allows catheter 32 to flex when angular pressure is applied to catheter 32 without constricting inner diameter D₂ of catheter 32. Flexible section 34 preferably can flex up to an angle of 180° from the point of insertion of the catheter into the patient at insertion site 38. Flow of fluids through catheter 32 remains constant after flexible section 34 is flexed.

The present invention has the advantage of increasing performance of an over-the-needle catheter and increasing patient comfort during use of the catheter. If a patient moves resulting in the catheter placement moving, the catheter of the present invention flexes to maintain constant flow of fluids through the catheter. The catheter is more stable after insertion into the patient and does not require extensive taping to prevent movement of the catheter. Accordingly, the catheter reduces the need for reinsertion of the catheter.

While the invention has been described with reference to the preferred embodiment, this description is not intended to be limiting. It will be appreciated by those of ordinary skill in the art that modifications may be made without departing from the spirit and scope of the invention.

## Claims

1. An over-the-needle catheter for inserting into a patient to allow fluid to flow into or from a body cavity of said patient comprising:
said catheter attached to a hollow catheter hub and including a flexible section; and
a hollow needle concentric with said catheter and extending from one end thereof for piercing said patient and inserting said catheter in place within the body of said patient;
said hollow needle movable into said hollow catheter hub during use of said catheter,
wherein said flexible section flexes during use of said catheter upon movement of said patient, thereby maintaining constant flow of said fluid through said catheter.

2. The over-the-needle catheter of claim 1 wherein said flexible section flexes up to about 180 degrees from the point of insertion of said catheter in said patient.

3. The over-the-needle catheter of claim 2 wherein said catheter has an inner diameter D₂ and said inner diameter D₂ of the catheter is substantially constant after flexing of said flexible section.

4. The over-the-needle catheter of claim 3 wherein said flexible section is formed by compression during molding of said catheter to form ridges in said catheter, said ridges and the portion of said catheter between said ridges expanding when said catheter is flexed.

5. The over-the-needle catheter of claim 4 wherein said flexible section is positioned adjacent the end of said catheter attached to said catheter hub.

6. The over-the-needle catheter of claim 1 wherein said catheter is integral with said catheter hub.

7. Acatheterforinserting into a patient to allow fluid to flow into or from a body cavity of said patient comprising:
said catheter attached to a hollow catheter hub and including a flexible section,
wherein said flexible section flexes during use of said catheter upon movement of said patient, thereby maintaining constant flow of said fluid through said catheter.

8. The catheter of claim 7 wherein said flexible section flexes up to about 180 degrees from the point of insertion of said patient.

9. The catheter of claim 8 wherein said catheter has an inner diameter D2 and said inner diameter D2 of the catheter is substantially constant afterflex- ing of said flexible section.

10. The catheter of claim 9 wherein said flexible section is formed by compression during molding of said catheter to form ridges in said catheter, said ridges and the portion of said catheter between said ridges expanding when said catheter is flexed.
